# EUROPÄISCHE PATENTSCHRIFT

(11) **EP 1 331 478 B1**
(45) Veröffentlichungstag und Bekanntmachung des Hinweises auf die Patenterteilung: **20.01.2016**
(21) Anmeldenummer: 03001639.8
(22) Anmeldetag: 24.01.2003
(51) Int. Cl.: G01N 27/417, G01N 27/419, G01N 27/407, G01N 33/00, F01N 11/00

(54) **Verfahren zur Bestimmung der NOx-Konzentration in Abgasen**
Method for the detection of the concentration of NOx in exhaust gas
Procédé pour déterminer la concentration de NOx dans des gaz d'échappement

(30) Priorität: 24.01.2002 DE 10202859
(43) Veröffentlichungstag der Anmeldung: 30.07.2003
(73) Patentinhaber: Volkswagen Aktiengesellschaft, 38440 Wolfsburg (DE)
(72) Erfinder: Hahn, Hermann, Dr.-Ing., 30175 Hannover (DE); Schlüter, Jens, 32351 Stemwede (DE)
(74) Vertreter: Pohlmann, Bernd Michael

(56) Entgegenhaltungen:
- EP-A- 0 841 562
- EP-A- 0 937 979
- EP-A- 0 995 986
- EP-A- 0 999 442
- EP-A- 1 026 501
- EP-A- 1 074 833
- DE-A- 10 049 685
- DE-A- 19 819 462
- US-A- 4 601 276
- US-B1- 6 290 829

## Beschreibung

Die Erfindung bezieht sich auf ein Verfahren gemäß dem Oberbegriff des unabhängigen Patentanspruchs.

Bei Kraftfahrzeugen mit modernen Verbrennungsmotoren, die im Mager- und Schichtladebetrieb einen geringeren Kraftstoffverbrauch aufweisen, ist zur Erfüllung der gesetzlichen Abgasvorschriften eine zusätzliche Nachbehandlung der Abgase zur Reduzierung von Stickoxyd-Emissionen notwendig. Bevorzugt werden zur Lösung dieses Problems NOx-Speicherkatalysatoren eingesetzt, zu deren Überwachung NOx-Sensoren verwendet werden. Um eine hohe Emissionsstabilität des Motors zu erreichen, ist eine möglichst hohe Genauigkeit bei der Messung der NOx-Konzentration im Abgas, insbesondere für eine präzise Regelung des Magerbetriebs und des Speicherzyklus des NOx-Katalysators erforderlich. Aus der EP 0892265 A1 ist in diesem Zusammenhang bereits ein Gas-Sensor für die Messung von Gasoxyden bekannt, bei dem Abgas zur Messung in ein Doppel-Messkammersystem geführt wird. Die Messkammern weisen für die Messung Nernst-Zellen auf. Während in der ersten Messkammer Sauerstoffmoleküle dem Gasgemisch entzogen werden, wird in der zweiten Messkammer das zu messende Gasoxyd, beispielsweise Stickoxyd, in Stickstoff und Sauerstoff zerlegt. Eine an die erste Kammer angelegte Pumpzellenspannung wird auf einen konstanten Wert geregelt, der einer konstanten Sauerstoffkonzentration in dieser Kammer entspricht.

Idealerweise zeigt das Ausgangssignal eines NOx-Sensors exakt die tatsächliche NOx-Konzentration des Abgases an. Insbesondere sollte ein NOx-Sensor unter Bedingungen ohne NOx-Emission ein NOx-Signal = 0 liefern. Dies ist beispielsweise von Bedeutung für die Regelung des Speicherzyklus und für die Diagnose eines NOx-Speicherkatalysators mit hoher Einspeicherfähigkeit im Magerbetrieb, bei dem stromab des Speicherkatalysators bei nichtgeschädigtem Speicherkatalysator keine NOx-Emissionen auftreten. Hierzu ist es bekannt, die Ausgangssignale eines NOx-Sensors zu korrigieren. So aus der DE 19819462 A1 ein Verfahren bekannt, bei dem die Ausgangssignale eines NOx-Sensors mit Hilfe von in Kennfeldern abgelegten Korrekturwerten korrigiert werden. Die Kennfelder sind insbesondere von der Sauerstoffkonzentration und deren Änderungsrate im Abgas sowie von der Temperatur des Sensors sowie des Abgases abhängig. Der Meßwert der NOx-Konzentration wird hierbei mit einem mulitplikativen Korrekturwert und einem additiven Korrekturwert korrigiert.

Aus der EP 1 077 375 A1 ist ferner ein Verfahren bekannt, bei dem die Stickoxydkonzentration in Abhängigkeit vom Wert der Sauerstoffkonzentration im Abgas und von der Änderungsrate der gemessenen Sauerstoffkonzentration korrigiert wird.

Aus der WO 99/57555 ist bereits ein Verfahren zur Bestimmung der NOx-Konzentration in einem Gas mittels eines NOx-Sensors mit zwei Messzellen bekannt, bei dem der Messwert der NOx-Konzentration mit einem multiplikativen Korrekturwert und einem additiven Korrekturwert korrigiert wird. Dabei sind beide Korrekturwerte aus einem Kennfeld entnommen, dass mindestens von einem Sauerstoffionenpumpstrom abhängt, mittels dessen eine Sauerstoffkonzentration in der zweiten Messzelle eingeregelt wird. Das Kennfeld wird dabei in einer Prüfstandsvermessung unter Variation von mindestens einem der folgenden Parameter bestimmt: NOx-Konzentration, Änderungsrate der NOx-Konzentration, O2-Konzentration, Änderungsrate der O2-Konzentration.

Gemäß dem Verfahren der EP 1077375 A1 wird ein Off-Set-Wert für das NOx-Signal berechnet. Ferner ist aus der WO 01/38864 A2 ein Verfahren zur Korrektur der durch Sauerstoff hervorgerufenen Messwertverfälschung an einem NOx-Sensor bekannt. Dabei wird ein Lambdawert des Abgases bestimmt, aus dem ein Messfehler der gemessenen NOx-Konzentration bestimmt wird.

Nachteilig an dem bekannten Stand der Technik ist, dass Serienstreuungen nur mit großem Aufwand korrigiert werden können. Ferner sind dynamische Abweichungen des NOx-Signals von dem exakten Messwert, die aus nichtstationären Fluktuationen des Sensor-Regelzustandes resultieren, nicht mit hinreichender Genauigkeit darstellbar.

Aufgabe der vorliegenden Erfindung ist daher die Schaffung eines Verfahrens, mit dem die Genauigkeit mit der die NOx-Konzentration im Abgas einer Brennkraftmaschine ermittelt wird erhöht werden kann.

Aus dem Dokument EP 1 026 501 A2 ist bereits eine Vorrichtung zur Messung von Gaskonzentrationen bekannt, mit dem eine Filterung von Sensorsignalen erfolgen kann. Mit der Filterung soll erreicht werden, dass die Sensorsignale durch mit messen unerwünschter Leckströme verfälscht werden. Eine Erfassung von Fluktuationen eines Signals in einem vorgegebenen Erfassungsinterwall findet nicht statt. Ein Sauerstoffsignal sowie ein Nox-Signal werden jeweils für sich mit festen Einstellungen gefiltert.

Aus dem Dokument EP 0 999 442 A2 ist ferner bekannt ein NOx-Signal durch ein gemessenes Sauerstoffsignal zu korrigieren, wobei von der Erkenntnis ausgegangen wird, das abhängig von der Gesamt-Sauerstoffkonzentration eine Verfälschung der NOx-Messung erfolgt. Eine Erfassung und Bewertung von Fluktuationen des Sauerstoffsignals über einen gewissen Zeitraum erfolgt nicht.

Diese Aufgabe wird erfindungsgemäß mit den Merkmalen des unabhängigen Anspruchs gelöst.

Der Erfindung liegt die Erkenntnis zu Grunde, dass die durch zeitliche Fluktuationen der Sauerstoffkonzentrationen im Abgas bewirkten Änderungen des NOx-Signals kurzzeitig höher sein können als sonstige stationäre Abweichungen und dass diese das NOx-Messergebnis verfälschenden Einflüsse durch eine Filterung des NOx-Signals beseitigt oder vermindert werden können.

Eine besonders einfache Erfassung besteht in der Verwendung von Lambdawertsignalen, die vom NOx-Sensor selbst zur Verfügung gestellt werden.

Um eine zeitliche Verlängerung der Korrekturwirkung des Zustandssignals auf das NOx-Signal zu erreichen, wird bei der Bildung des Zustandssignals eine Verzögerungszeit berücksichtigt.

Bevorzugt wird zur Filterung eine Tiefpassfilterung eingesetzt, da hiermit hochfrequente bzw. kurzzeitige Fluktuationen des NOx-Signals reduziert werden können. Eine besonders einfache Filterung wird durch Anwendung einer zeitlich gleitenden Mittelung mit einem Filterfaktor erreicht. Hierbei ist es zweckmäßig, den Filterfaktor in Abhängigkeit von einer zeitlichen Änderungsrate eines Lambdawertsignals zu wählen.

Eine weitere Verbesserung der Korrektur kann erreicht werden, wenn der Filterfaktor in Abhängigkeit von weiteren den Sensor-Regelzustand beeinflussenden Größen, insbesondere einer Abgasgeschwindigkeit, einer Sensortemperatur und / oder einer Abgastemperatur gewählt wird.

In weiteren Ausführungsformen der Erfindung werden in Abhängigkeit von vorgegebenen Betriebsparametern der Brennkraftmaschine Werte des zu filternden NOx-Signals und / oder eine Freigabe der Filterung beeinflusst.

Weitere Verbesserungen der Korrektur lassen sich erreichen, wenn das NOx-Signal vor und / oder nach der Filterung in Abhängigkeit von dem Zustandssignal einer Korrektur mit einer additiven und / oder multiplikativen Korrekturwert und / oder einer Adaption unterworfen wird.

Nachfolgend wird die Erfindung anhand eines in Zeichnungen dargestellten Ausführungsbeispiels näher beschrieben, aus dem sich auch unabhängig von der Zusammenfassung in den Patentansprüchen weitere Merkmale, Einzelheiten und Vorteile der Erfindung ergeben.

Es zeigen in schematischer Darstellung:
- Figur 1: eine Brennkraftmaschine mit zugeordneter Abgasanlage,
- Figur 2: ein NOx-Sensor,
- Figur 3: den zeitlichen Verlauf eines Lambdasignalwerts, eines NOx-Signalwerts und eines Delta-Lambda-Wertes.

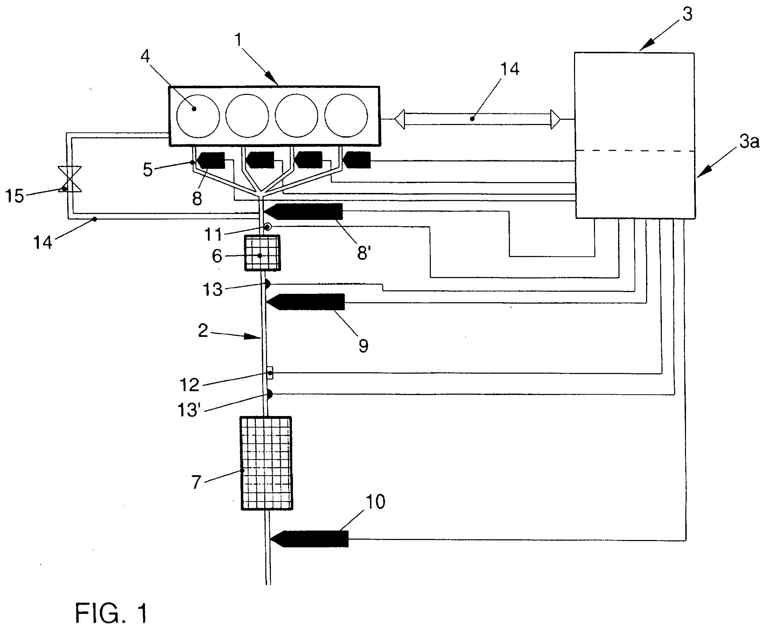

Figur 1 zeigt zur Veranschaulichung verschiedener Einbaulagen von NOx-Sensoren in schematischer Darstellung eine Brennkraftmaschine 1, beispielsweise ein magerlauffähiger Ottomotor oder eine Dieselbrennkraftmaschine, mit einer Abgasanlage 2 und einem Motorsteuergerät 3, vorzugsweise zum Betrieb eines Kraftfahrzeugs. Die Brennkraftmaschine 1 weist eine Anzahl von Zylinderbänken 4 auf (entsprechende Komponenten sind nur mit einem Bezugszeichen versehen), denen jeweils ein eigener Abgaspfad 5 nachgeschaltet ist. In der Abgasanlage 2 ist zur Konvertierung von schädlichen oder unerwünschten Komponenten des Abgases in andere Komponenten eine Abgasreinigungsvorrichtung mit einem Vorkatalysator 6 und einem Hauptkatalysator 7 angeordnet. Vorzugsweise ist der Vorkatalysator 6 als 3-Wege-Katalysator und der Hauptkatalysator 7 als NOx-Speicherkatalysator ausgebildet. Stromabwärts der Zylinderbänke 4 sind in den Abgaspfaden 5 NOx-Sensoren 8 angeordnet, mit denen die NOx-Konzentration des durch die Abgasanlage 2 geführten Abgases der Brennkraftmaschine 1 gemessen werden kann. Stromaufwärts des Vorkatalysators 6 ist zudem ein weiterer NOx-Sensor 8' angeordnet. In einem Bereich der Abgasanlage 2 zwischen dem Vorkatalysator 6 und dem Hauptkatalysator 7, stromabwärts des Vorkatalysators 6 und stromaufwärts des Hauptkatalysators 7, ist ein weiterer NOx-Sensor 9 angeordnet. Ein weiterer Sensor 10 ist stromabwärts des Hauptkatalysators 7 in der Abgasanlage 2 angeordnet. Es versteht sich von selbst, dass bei einer Abgasreinigungsvorrichtung mit mehreren Teilen, Sensoren stromauf oder stromab der jeweiligen Teile angeordnet sein können.

Zusätzlich zu den erwähnten Sensoren sind stromaufwärts und stromabwärts des Vorkatalysators 6 und stromaufwärts des Hauptkatalysators 7 Lambda-Sonden 11 bzw. 12 sowie zur Ermittlung der Betriebstemperatur der Katalysatoreinrichtungen Temperatursensoren 13, 13' vorgesehen. Es versteht sich von selbst, dass alternativ oder zusätzlich weitere Temperatursensoren zur Messung der Betriebestemperatur der Abgasreinigungsvorrichtung oder von Teilen dieser vorgesehen sein können. Zur Abgasrückführung weist die Brennkraftmaschine 1 eine Abgasrückführeinrichtung 14 mit einem steuerbaren Ventil 15 auf.

Das Motorsteuergerät 3 erfasst in an sich bekannter Weise über nicht dargestellte weitere Sensoren Betriebsparameter der Brennkraftmaschine 1, wie beispielsweise Drosselklappenstellung, Abgasrückführungsrate, Zündzeitpump, Einspritzzeitpump von Vor- / Haupt- / Nacheinspritzungen, Einspritzdruck, Tumble-Klappenstellung, Ladedruck, Phasensteller der Nockenwelle, Drehzahl, Fahrpedalstellung, Last, Fahrgeschwindigkeit und dergleichen, und kann diese über (nicht dargestellte) Stellglieder gegebenenfalls beeinflussen, wobei zur Kommunikation zwischen dem Motorsteuergerät 3 und den Sensoren bzw. Stellgliedern ein Kabelsystem 14 oder dergleichen vorgesehen ist. Ferner umfasst das Motorsteuergerät 3 eine Lambda-Regeleinrichtung zur Regelung der Sauerstoffkonzentration im Abgas bzw. des Lambda-Werts. Das Motorsteuergerät 3 erhält von den NOx-Sensoren NOx-Signale mit denen die NOx-Rohemissionen der Brennkraftmaschine 1 bzw. die NOx-Konzentration stromabwärts der Katalysatoreinrichtungen 6 und / oder 7 ermittelt werden können.

Der NOx-Speicherkatalysator 7 wird üblicherweise in einem Speicherzyklus betrieben, der zumindest einen üblicherweise langsamen Absorptionsmodus und einen schnelleren Regenerationsmodus umfasst. Die absorptive Speicherung erfolgt dabei bei einem Lambda-Wert >1, die Ausspeicherung zu einem späteren Zeitpump bei einem Lambda-Wert <1 oder =1.

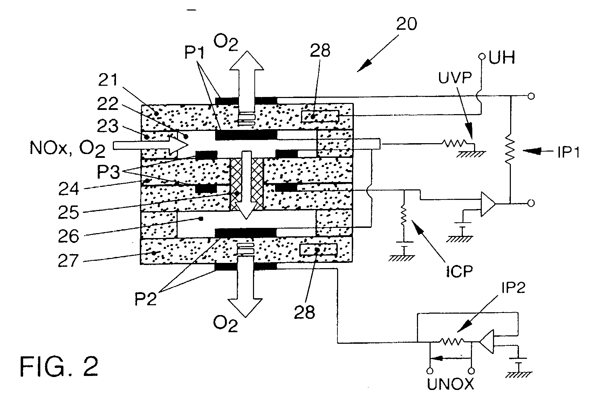

Der in Figur 2 dargestellte NOx-Sensor 20 zur Messung der NOx-Konzentration im Abgas besteht aus einer Keramik 30 und ist als Doppelkammersensor mit einer ersten und zweiten Messkammer ausgebildet. Über eine Diffusionsbarriere kann ein Teil des Abgases mit NOx-, 02- und weiteren Komponenten die erste Messkammer erreichen. Die Messkammer weist eine nach dem Nernst-Prinzip arbeitende Sauerstoff-Messpumpzelle mit Pumpelektroden P1 auf. Mittels letzterer kann der Sauerstoffgehalt in der Messkammer verändert werden. Über eine- weitere Diffusionsbarriere gelangt Abgas in die zweite Messkammer. Diese weist eine ebenfalls nach dem Nernst-Prinzip arbeitende NOx-Messpumpzelle mit Pumpelektroden P2 auf. Die im Abgas enthaltenen StickOxyde werden durch ein spezielles Material der inneren P2 Elektroden katalytisch in die Komponenten N2 und 02 zerlegt. Zur Kalibrierung des Systems wird, eine 02-Referenzzelle mit Elektroden P3 verwendet.

An die Sauerstoff-Messpumpzelle wird eine Pumpspannung UVS angelegt. Der Strom ICP wird so geregelt, dass ein konstanter, vorzugsweise stöchiometrischer Wert der Sauerstoff-Konzentration in der ersten Messkammer resultiert. Aus dem Pumpstrom IP1 wird ein Breitband-Lambdawert U02 berechnet. Die Spannung UVP entspricht einem Lambdasprungsignal. Über den Pumpzellenstrom IP2 der Messkammer kann ein der NOx-Konzentration im Abgas entsprechendes Spannungssignal UNOX ermittelt werden.

Um eine für eine NOx -Messung erforderliche Mindesttemperatur von z.B. 740 Grad zu gewährleisten, weist der NOx-Sensor 20 Heizelemente auf, denen eine Heizspannung UH von einer nicht dargestellten Heizungseinrichtung zugeführt wird ist. Eine zugeordnete nicht dargestellte Temperatur-Messeinrichtung gibt ein Temperatursignal ab, aus dem die NOx-Sensortemperatur ermittelbar wird.

Die Einregelung der Sauerstoffmesspumpzelle 21 erfolgt typischerweise über kürzere Zeiten (<= 1 sec) als die der NOx-Messpumpzelle 27 (3-4 sec). Dabei hängt das NOx-Signal von vielen Einflussgrößen wie beispielsweise Temperaturschwankungen am Sensor, stationärer Gasdruck am Sensor, Sauerstoffkonzentration, NH3-Emission oder dergleichen ab. Der Effekt dieser Einflussgrößen kann in an sich bekannter Weise kompensiert werden, sobald es sich um stationäre Störungen handelt. Jedoch können die durch nicht stationäre Störungen oder Fluktuationen erzeugten Abweichungen des NOx-Signals vom exakten Messwert kurzzeitig größer sein als die durch stationäre Störungen bewirkten, da bei diesen dynamischen Vorgängen die Sauerstoffmesspumpzelle 21 und die NOx-Messpumpzelle 27 nicht ins Gleichgewicht eingeregelt sind. Nicht stationäre Fluktuationen des Sensor-Regelzustandes werden vor allem durch Fluktuationen in der Abgaszusammensetzung bewirkt. Weitere den Sensor-Regelzustand beeinflussende Größen sind vor allem die Abgasgeschwindigkeit in Sensornähe, die Sensortemperatur und die Abgastemperatur. Da der Sauerstoffzustrom ICP der Sauerstoffmesspumpzelle 21 als Integral des Potentialgefälles über die gesamte Zelle eingestellt wird, kann es im Falle dynamischer Änderungen der Abgaszusammensetzung, insbesondere bei Änderungen in der Sauerstoffkonzentration in der Zelle zu gegenüber dem Regel-Sollwert höheren oder niedrigeren Sauerstoffdurchlässen in die NOx-Messpumpzelle 27 kommen.

Erfindungsgemäß wird für ein Erfassungsintervall der nicht stationäre Sensor-Regelungszustand erfasst. Das Erfassungsintervall wird vorzugsweise größer oder gleich einem typischen Einregelungszeitintervall gewählt. Vorzugsweise werden dazu Fluktuationen der Abgaszusammensetzung, insbesondere der Sauerstoffkonzentrationsfluktuationen erfasst. Die Sauerstoffkonzentrationsfluktuationen können aus dem Signal UVP und IP1 bestimmt werden. Bevorzugt wird dazu der Wert der Änderungsrate des Lambdawertsignals berechnet und als die Größe der Fluktuationen charakterisierendes Zustandssignal verwendet. In Abhängigkeit von dem Zustandssignal wird das NOx-Signal zur Bildung eines Signals mit reduzierten Fluktuationen gefiltert und zur Korrektur des NOx-Signals herangezogen. Hierzu weist das Motorsteuergerät 3 eine Filtereinrichtung 3a auf. Im einfachsten Fall wird das gefilterte NOx-Signal als korrigiertes NOx-Signal verwendet.

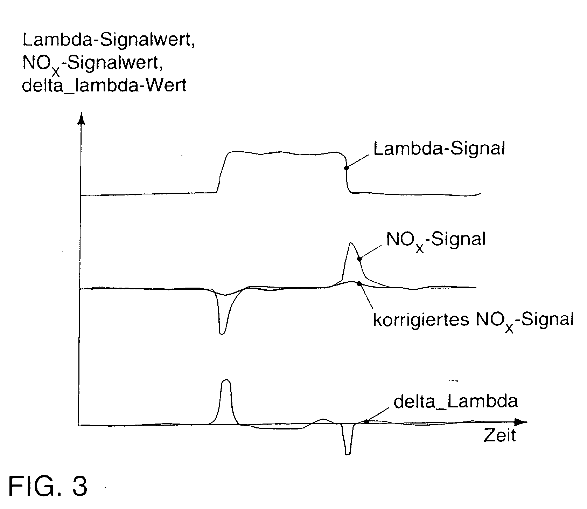

In Figur 3 sind der zeitliche Verlauf eines Lambdasignalwertes, eines NOx-Signals, eines durch Filterung gebildeten korrigierten NOx-Signals und einer zeitlichen Änderungsrate (zeitliche Ableitung) des Lambdasignals gegenüber der Zeit aufgetragen. Bei dem Lambdasignal handelt es sich um ein Zweipunkt-Lambdasignal. Wie ersichtlich ist, korrespondiert zu der Änderung des Lambdasignalwerts eine Änderung des NOx-Signals. Einem Peak in der zeitlichen Änderungsrate des dargestellten Lambdasignals ist ein Peak im NOx-Signal zugeordnet, bei dem es sich jedoch um ein Artefakt gegenüber dem exakten Wert der NOx-Konzentration handelt. Das korrigierte NOx-Signal zeigt in dem zeitlichen Bereich der Änderung des Lambdasignals zwar auch einen Peak, der jedoch wesentlich geringer ausgebildet ist. Ohne die Korrektur kommt es üblicherweise bei einer Änderung der Sauerstoffkonzentration des Abgases hin zu höheren Werten zu beträchtlichen Unterschwingern im NOx-Signal. Ändert sich die Sauerstoffkonzentration zu niedrigeren Werten, kommt es umgekehrt zu Überschwingern im NOx-Signal.

Die Filterung des NOx-Signals erfolgt vorzugsweise gemäß folgender Vorschrift: Aktuell gefilterter Wert = vorheriger gefilterter Wert + (aktueller NOx-Signalwert-vorheriger Filterwert) * F, F = Filterfaktor. Allerdings sind auch andere Filterfunktionen, insbesondere Tiefpass- oder Bandpassfilter erfindungsgemäß einsetzbar.

Der Filterfaktor F wird in Abhängigkeit von dem Wert des Zustandssignals und damit von der Größe der nicht stationären Fluktuationen gewählt. Vorzugsweise wird für den Filterfaktor die Form F = 1 - Konstante * Delta-Lambda oder = Konstante / Delta-Lambda gewählt. Der Filterfaktor F ist dabei auf einen Wert <= 1 beschränkt. Delta-Lambda bezeichnet hier die zeitliche Änderungsrate eines Lambdawertsignals. Alternativ ist der Filterwert F durch ein Kennfeld in Abhängigkeit von der zeitlichen Änderungsrate eines Lambdawerts vorgegeben. Im Allgemeinen wird in einem Betrieb mit geringen nicht stationären Fluktuationen ein Filterfaktor F nahe 1 gewählt, womit der aktuelle NOx-Signalwert nur wenig gefiltert wird. Mit zunehmender Größe der nicht stationären Fluktuationen wird der Filterwert kleiner gesetzt, was zu einer stärkeren Filterwirkung und Reduktion von Fluktuationen im gefilterten NOx-Signal führt. Um den Einfluss weiterer dem Sensor-Regelzustand beeinflussenden Größen zu berücksichtigen, kann der Filterfaktor auch in Abhängigkeit von diesen weiteren Größen, insbesondere der Abgasgeschwindigkeit, der Sensortemperatur oder der Abgastemperatur variiert werden. Beispielsweise hier kann eine entsprechende Eingangsgröße für das Kennfeld verwendet werden.

In Ergänzung zu den oben geschilderten Maßnahmen wird bei der Bildung des Zustandssignals eine Verzögerungszeit berücksichtigt. Hiermit wird dem Umstand Rechnung getragen, dass die Einregelung der NOx-Messpumpzelle länger dauert als die Einregelung der Sauerstoffmesspumpzelle. Dementsprechend kann beispielsweise der Wert des Zustandssignals gefiltert oder gedämpft und so in seiner zeitlichen Wirksamkeit verlängert werden.

Ferner kann eine zeitliche Verlängerung durch Vorgabe einer festen oder in Abhängigkeit von anderen Größen, beispielsweise motorischen und / oder Sensorgrößen festgelegten Verzögerungszeit realisiert werden.

Für den Fall, dass Betriebsbereiche der Brennkraftmaschine anliegen, in denen große Quereinflüsse des NOx-Signals verändern oder bestimmen, ist es zweckmäßig, ein fiktives NOx-Signal auf vorgegebene Werte zu setzen und zu filtern. Dies kann z.B. ein stöchiometrischer oder fetter Motorbetrieb sein, oder auch Schubabschaltungsphasen, in denen der Motor geschleppt wird ohne dass eine Verbrennung erfolgt.

Ferner ist es zweckmäßig, eine Freigabe der Filterung vorzusehen, so dass nur bei bestimmten vorgegebenen Betriebszuständen eine Filterung erfolgt.

Um zu einer noch weiter erhöhten Genauigkeit bei der Bestimmung der NOx-Konzentration zu gelangen, kann das NOx-Signal vor und / oder nach der Filterung in Abhängigkeit von dem Wert des Zustandssignals einer Korrektur mit einem additiven und / oder multiplikativen Korrekturwert unterworfen werden. Hiermit kann, wie an sich bekannt ist, der Einfluss von stationären Veränderungen berücksichtigt werden. Ebenso kann eine Adaption der Filterung während eines vorgegebenen Adaptionsintervalls erfolgen. Auf der Grundlage des NOx-Signals während eines vorgegebenen Adaptionsintervalls wird hierbei das korrigierte vorzugsweise gefilterte NOx-Signal variiert und an den exakten NOx-Wert angepasst.

## Patentansprüche

1. Verfahren zur Bestimmung der NOx-Konzentration im Abgas einer Brennkraftmaschine mittels eines in der Abgasanlage der Brennkraftmaschine angeordneten NOx-Sensors, wobei nichtstationäre Fluktuationen eines Sensor-Regelzustandes erfasst werden, **dadurch gekennzeichnet, dass**
- für ein vorgegebenes Erfassungsintervall zeitliche Fluktuationen der Sauerstoffkonzentration im Abgas erfasst werden;
- ein die Größe der Fluktuationen charakterisierendes Zustandssignal gebildet wird;
das Zustandssignal in Abhängigkeit von dem Wert der zeitlichen Änderungsrate eines Lambdawertsignals gewählt wird.
- in Abhängigkeit von dem Zustandssignal eine Filterung des NOx-Signals zur Bildung eines Signals mit reduzierten Fluktuationen erfolgt, welches zur Korrektur des Nox-Signals herangezogen wird oder direkt als korrigiertes Signal verwendet wird.

2. Verfahren nach Anspruch 1, **dadurch gekennzeichnet, dass** zur Erfassung der Fluktuationen der Sauerstoffkonzentration ein Lambdawertsignal, ein Zweipunkt- und / oder ein Breitbandlambdasignal des NOx-Sensors verwendet wird.

3. Verfahren nach einem der vorhergehenden Ansprüche, **dadurch gekennzeichnet, dass** eine Verzögerungszeit bei der Bildung des Zustandssignals berücksichtigt wird.

4. Verfahren nach einem der vorhergehenden Ansprüche, **dadurch gekennzeichnet, dass** einem zunehmenden Wert des Zustandssignals eine stärkere und einem abnehmenden Wert des Zustandssignals eine geringere Reduktion der Fluktuation des NOx-Signals entspricht.

5. Verfahren nach einem der vorhergehenden Ansprüche, **dadurch gekennzeichnet, dass** die Filterung eine Tiefpassfilterung oder Bandpassflterung des NOx-Signals ist.

6. Verfahren nach einem der vorhergehenden Ansprüche, **dadurch gekennzeichnet, dass** die Filterung eine zeitliche gleitende Mittelung des NOx-Signals der Form: aktueller gefilterter Wert = vorheriger gefilterter Wert + (aktueller NOx-Signalwert-vorheriger gefilterter Wert) * F, wobei F <= 1 ein Filterfaktor ist.

7. Verfahren nach einem der vorhergehenden Ansprüche, **dadurch gekennzeichnet, dass** die Filterung oder der Filterfaktor in Abhängigkeit von einer zeitlichen Änderungsrate eines Lambdawertsignals verändert wird.

8. Verfahren nach einem der vorhergehenden Ansprüche, **dadurch gekennzeichnet, dass** die Filterung oder der Filterfaktor gemäß einem Kennfeld in Abhängigkeit von einer zeitlichen Änderungsrate eines Lambdawertsignals variiert wird.

9. Verfahren nach einem der vorhergehenden Ansprüche, **dadurch gekennzeichnet, dass** die Filterung oder der Filterfaktor in Abhängigkeit von weiteren den Sensor-Regelzustand beeinflussenden Größen, einer Abgasgeschwindigkeit, einer Sensor-Temperatur und / oder einer Abgastemperatur variiert wird.

10. Verfahren nach einem der vorhergehenden Ansprüche, **dadurch gekennzeichnet, dass** in Abhängigkeit von vorgegebenen Betriebsparametern der Brennkraftmaschine das zu filternde NOx-Signal auf vorgegebene Werte gesetzt wird.

11. Verfahren nach einem der vorhergehenden Ansprüche, **dadurch gekennzeichnet, dass** in Abhängigkeit von vorgegebenen Betriebsparametern der Brennkraftmaschine eine Freigabe der Filterung vorgesehen ist, so dass nur bei bestimmten vorgegebenen Betriebszuständen eine Filterung erfolgt.

12. Verfahren nach einem der vorhergehenden Ansprüche, **dadurch gekennzeichnet, dass** das NOx-Signal vor und / oder nach der Filterung in Abhängigkeit von dem Wert des Zustandssignals einer Korrektur mit einem additiven und / oder multiplikativen Korrekturwert unterworfen wird.

13. Verfahren nach einem der vorhergehenden Ansprüche, **dadurch gekennzeichnet, dass** eine Adaption der Filterung auf der Grundlage des NOx-Signals während eines vorgegebenen Adaptionsintervalls erfolgt, wobei das gefilterte NOx-Signal variiert und an den exakten NOx-Wert angepasst wird.

## Claims

1. Method for determining the NO_{X} concentration in the exhaust gas of an internal combustion engine by means of an NO_{X} sensor which is arranged in the exhaust system of the internal combustion system, wherein non-steady-state fluctuations in a sensor control state are detected,
**characterized in that**
- fluctuations in the oxygen concentration in the exhaust gas over time are detected for a predefined detection interval;
- a status signal which characterizes the size of the fluctuations is formed; the status signal is selected as a function of the value of the rate of change of a lambda value signal over time;
- filtering of the NO_{X} signal is carried out as a function of the status signal in order to form a signal with reduced fluctuations, which signal is used to correct the NO_{X} signal or is employed directly as a corrected signal.

2. Method according to Claim 1, **characterized in that** a lambda value signal, a two point signal and/or a broadband lambda signal of the NO_{X} sensor is employed to detect the fluctuations in the oxygen concentration.

3. Method according to one of the preceding claims, **characterized in that** a delay time is taken into account in the formation of the status signal.

4. Method according to one of the preceding claims, **characterized in that** a relatively large reduction in the fluctuation of the NO_{X} signal corresponds to an increasing value of the status signal, and a relatively small reduction in the fluctuation of the NO_{X} signal corresponds to a decreasing value of the status signal.

5. Method according to one of the preceding claims, **characterized in that** the filtering is low-pass filtering or bandpass filtering of the NO_{X} signal.

6. Method according to one of the preceding claims, **characterized in that** the filtering is the formation of sliding averages of the NO_{X} signal over time in the form: current filtered value = previous filtered value + (current NO_{X} signal value - previous filtered value) *F, where F <= 1 is a filter factor.

7. Method according to one of the preceding claims, **characterized in that** the filtering or the filter factor is changed as a function of a rate of change of a lambda value signal over time.

8. Method according to one of the preceding claims, **characterized in that** the filtering or the filter factor is varied in accordance with a characteristic diagram as a function of a rate of change of a lambda value signal over time.

9. Method according to one of the preceding claims, **characterized in that** the filtering or the filter factor is varied as a function of further variables, an exhaust gas speed, a sensor temperature and/or an exhaust gas temperature, which influence the sensor control state.

10. Method according to one of the preceding claims, **characterized in that** the NO_{X} signal which is to be filtered is set to predefined values as a function of predefined operating parameters of the internal combustion engine.

11. Method according to one of the preceding claims, **characterized in that** enabling of the filtering is provided as a function of predefined operating parameters of the internal combustion engine, with the result that filtering takes place only in the case of certain predefined operating states.

12. Method according to one of the preceding claims, **characterized in that** the NO_{X} signal is subjected to correction with an additive and/or multiplicative correction value as a function of the value of the status signal before and/or after the filtering.

13. Method according to one of the preceding claims, **characterized in that** the filtering is adapted on the basis of the NO_{X} signal during a predefined adaptation interval, wherein the filtered NO_{X} signal is varied and adapted to the precise NO_{X} value.

## Revendications

1. Procédé de détermination de la concentration en NOₓ dans les gaz d'échappement d'un moteur à combustion interne au moyen d'un capteur de NOₓ disposé dans l'installation de gaz d'échappement du moteur à combustion interne, dans lequel des fluctuations non stationnaires d'un état de régulation du capteur sont détectées,
**caractérisé en ce que**
des fluctuations de la concentration en oxygène dans les gaz d'échappement sont détectées en fonction du temps dans un intervalle de détection prédéterminé,
**en ce qu'**un signal d'état caractérisant la taille des fluctuations est formé,
**en ce que** le signal d'état est sélectionné en fonction de la valeur de la vitesse de modification d'un signal de valeur lambda et
**en ce qu'**un filtrage du signal de NOₓ en fonction du signal d'état est effectué pour former un signal à fluctuation réduite qui intervient dans la correction du signal NOₓ ou qui est utilisé directement comme signal corrigé.

2. Procédé selon la revendication 1, **caractérisé en ce qu'**un signal de valeur lambda, un signal lambda en deux points et/ou un signal lambda en large bande du capteur de NOₓ sont utilisés pour détecter les fluctuations de la concentration en oxygène.

3. Procédé selon l'une des revendications précédentes, **caractérisé en ce qu'**une durée de retard est prise en compte dans la formation du signal d'état.

4. Procédé selon l'une des revendications précédentes, **caractérisé en ce qu'**une réduction plus forte de la fluctuation du signal NOₓ correspond à une valeur croissante du signal d'état et une réduction moindre de la fluctuation du signal NOₓ correspond à une valeur décroissante du signal d'état.

5. Procédé selon l'une des revendications précédentes, **caractérisé en ce que** le filtrage est un filtrage passe-bas ou un filtrage passe-bande du signal NOₓ.

6. Procédé selon l'une des revendications précédentes, **caractérisé en ce que** le filtrage est la formation d'une moyenne glissante du signal NO_{X} en fonction du temps, sous la forme : valeur filtrée actuelle = valeur filtrée antérieure + (valeur du signal NOₓ - valeur filtrée précédente) * F, F <= 1 étant un facteur de filtrage.

7. Procédé selon l'une des revendications précédentes, **caractérisé en ce que** le filtrage ou le facteur de filtrage sont modifiés en fonction de la vitesse de modification d'un signal de valeur lambda.

8. Procédé selon l'une des revendications précédentes, **caractérisé en ce que** le filtrage ou le facteur de filtrage sont modifiés selon un champ de caractéristiques en fonction de la vitesse de modification d'un signal de valeur lambda.

9. Procédé selon l'une des revendications précédentes, **caractérisé en ce que** le filtrage et le facteur de filtrage sont modifiés en fonction d'autres grandeurs qui agissent sur l'état de régulation du capteur, la vitesse de gaz d'échappement, la température du capteur et/ou la température des gaz d'échappement.

10. Procédé selon l'une des revendications précédentes, **caractérisé en ce que** le signal NO_{X} à filtrer est fixé à des valeurs prédéterminées en fonction de paramètres de fonctionnement prédéterminés du moteur à combustion interne.

11. Procédé selon l'une des revendications précédentes, **caractérisé en ce que** l'activation du filtrage est prévue de telle sorte que le filtrage n'ait lieu que dans des états de fonctionnement définis et prédéterminés en fonction de paramètres prédéterminés de fonctionnement du moteur à combustion interne.

12. Procédé selon l'une des revendications précédentes, **caractérisé en ce que** le signal NOₓ subit une correction à l'aide d'une valeur de correction additive et/ou multiplicative avant et/ou après le filtrage, en fonction de la valeur du signal d'état.

13. Procédé selon l'une des revendications précédentes, **caractérisé en ce qu'**une adaptation du filtrage s'effectue sur la base du signal NO_{X} pendant un intervalle d'adaptation prédétermine, le signal NOₓ filtré étant modifié et adapté à la valeur exacte des NOₓ.
